(19) Europäisches Patentamt / European Patent Office / Office européen des brevets

(11) **EP 4 031 281 B1**

(12) **FASCICULE DE BREVET EUROPEEN**

(45) Date de publication et mention de la délivrance du brevet:
**21.06.2023 Bulletin 2023/25**

(21) Numéro de dépôt: **20820488.3**

(22) Date de dépôt: **18.09.2020**

(51) Classification Internationale des Brevets (IPC):
**B01J 23/83** *(2006.01)*   **B01J 23/755** *(2006.01)*
**B01J 35/02** *(2006.01)*   **B01J 35/00** *(2006.01)*
**B01J 37/04** *(2006.01)*   **B01J 37/34** *(2006.01)*
**C07C 1/12** *(2006.01)*   **C10G 2/00** *(2006.01)*
**B01J 23/745** *(2006.01)*   **C07C 1/04** *(2006.01)*
**B01J 8/00** *(2006.01)*

(52) Classification Coopérative des Brevets (CPC):
(C-Sets disponibles)
**B01J 23/755; C07C 1/12; C10G 2/50;** B01J 8/001;
B01J 23/745; B01J 2208/00433; C07C 2523/745;
C07C 2523/755                          (Cont.)

(86) Numéro de dépôt international:
**PCT/FR2020/051625**

(87) Numéro de publication internationale:
**WO 2021/053306 (25.03.2021 Gazette 2021/12)**

(54) **PROCÉDÉ DE CATALYSE HÉTÉROGÈNE METTANT EN OEUVRE UN MATÉRIAU FERROMAGNÉTIQUE CHAUFFÉ PAR INDUCTION MAGNÉTIQUE**

VERFAHREN ZUR HETEROGENEN KATALYSE UNTER VERWENDUNG EINES DURCH MAGNETISCHE INDUKTION ERHITZTEN FERROMAGNETISCHEN MATERIALS

METHOD FOR THE HETEROGENEOUS CATALYSIS USING A FERROMAGNETIC MATERIAL HEATED BY MAGNETIC INDUCTION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.09.2019 FR 1910345**

(43) Date de publication de la demande:
**27.07.2022 Bulletin 2022/30**

(73) Titulaires:
• **Institut National des Sciences Appliquées de Toulouse**
  **31077 Toulouse Cedex 4 (FR)**
• **Centre national de la recherche scientifique**
  **75016 Paris (FR)**

(72) Inventeurs:
• **MARBAIX, Julien**
  **33300 BORDEAUX (FR)**
• **KERROUX, Pauline**
  **22700 SAINT QUAY PERROS (FR)**
• **KALE, Sumeet**
  **PUNE 411043 (IN)**
• **ASENSIO REVERT, Juan Manuel**
  **31400 TOULOUSE (FR)**
• **FAURE, Stéphane**
  **31140 LAUNAGUET (FR)**
• **MILLE, Nicolas**
  **31400 TOULOUSE (FR)**
• **CARREY, Julian**
  **31400 TOULOUSE (FR)**
• **SOULANTIKA, Aikaterini**
  **31810 CLERMONT LE FORT (FR)**
• **CHAUDRET, Bruno**
  **31320 VIGOULET AUZIL (FR)**

(74) Mandataire: **Ipsilon**
  **Le Centralis**
  **63, avenue du Général Leclerc**
  **92340 Bourg-la-Reine (FR)**

EP 4 031 281 B1

(56) Documents cités:
  **WO-A1-2015/140620**    **FR-A1- 3 045 412**
  **US-A- 4 359 379**      **US-A1- 2017 348 454**
  **US-A1- 2019 144 376**

(52) Classification Coopérative des Brevets (CPC):
  (Cont.)

  C-Sets
  **C07C 1/12, C07C 9/04**

**Description**

DOMAINE DE L'INVENTION

La présente invention concerne le domaine de la catalyse hétérogène, notamment un procédé de catalyse hétérogène gaz-solide comprenant la mise en contact d'au moins un réactif gazeux et d'un composé solide catalytique disposé sur un support.

De très nombreux procédés font appel à une catalyse hétérogène. Ces procédés de catalyse requièrent une étape de chauffage, parfois à haute température, pour la réalisation de la réaction, et sont donc coûteuses et très consommatrices d'énergie. La recherche s'est donc orientée vers des solutions plus économiques et notamment vers des réactions moins gourmandes en énergie.

ETAT DE LA TECHNIQUE ANTERIEURE

Parmi ces solutions il a été proposé dans le document WO2014/162099 un procédé de catalyse hétérogène dans lequel le chauffage est réalisé par induction magnétique pour atteindre la température nécessaire à la réaction. Plus particulièrement dans ce procédé, le réactif est mis en contact avec une composition catalytique qui comprend un composant nano-particulaire ferromagnétique dont la surface est constituée au moins en partie d'un composé catalyseur de ladite réaction, ledit composant nano-particulaire étant chauffé par induction magnétique pour atteindre la plage de température souhaitée. Ce chauffage peut être réalisé au moyen d'un inducteur de champ externe au réacteur. Dans ce système, les nanoparticules sont chauffées par leur propre moment magnétique, permettant le démarrage de la réaction catalytique. Le chauffage est donc initié au sein même du réacteur, de manière rapide avec un apport d'énergie minimal. Il en découle des économies substantielles.

Le document US 2017/348454A1 divulgue un purificateur d'air comprenant un convertisseur catalytique, le convertisseur catalytique comprenant un matériau catalytiquement actif et un matériau chauffable en contact thermique avec ledit matériau catalytiquement actif, matériau actif, dans lequel le matériau chauffable peut être chauffé par un ou plusieurs d'un champ électrique alternatif et d'un champ magnétique alternatif, le purificateur d'air comprenant en outre un générateur de champ, configuré sans contact électrique avec le matériau chauffable et configuré pour chauffer pendant le fonctionnement du purificateur d'air le matériau chauffable par un ou plusieurs parmi le champ électrique alternatif et le champ magnétique alternatif.

Le document US 4 359 379A divulgue un procédé de craquage catalytique fluide d'une huile de départ, qui comprend le soutirage d'une partie des particules de catalyseur circulant dans une unité de craquage catalytique fluide, envoyer les particules de catalyseur prélevées au moyen d'un fluide porteur choisi dans le groupe constitué par l'air, l'azote, la vapeur et leurs mélanges à une vitesse de 0,01 à 100 mètres/seconde dans une concentration de particules de 0,01 à 500 g/litre à un niveau élevé séparateur magnétique à gradient dans lequel une matrice ferromagnétique est placée dans un champ magnétique élevé uniforme pour générer un gradient magnétique élevé autour de la matrice, séparant ainsi les particules de catalyseur retirées en un groupe de particules rendues magnétiques par le dépôt d'au moins un métal choisi parmi le groupe composé de nickel, vanadium, fer et cuivre qui sont contenus dans l'huile de départ et un groupe de particules non magnétiques; et renvoyer les particules non magnétiques à l'unité de craquage catalytique fluide pour réutilisation.

Le document US 2019/144376A divulgue un procédé de synthèse d'un nitrile par réaction catalysée endothermique de l'ammoniac avec un hydrocarbure à l'aide d'un chauffage obtenu en faisant passer un courant alternatif dans une bobine métallique, la réaction endothermique entre l'ammoniac et l'hydrocarbure a lieu dans un réacteur à chauffage inductif direct dans la zone de réaction.

Le document WO2015/140620A1 divulgue un procédé dans lequel tout en réalisant une réaction chimique, des particules du catalyseur comprenant un matériau ferromagnétique sont mises en oscillation par le champ magnétique oscillant avec une fréquence supérieure à 0,1 Hz et une induction de champ magnétique supérieure à 0,01 mT.

Le document FR 3 045 412A1 divulgue des nanoparticules de fer (particulièrement adaptées pour être utilisées pour l'hyperthermie et pour catalyser les réactions de Sabatier et Fischer-Tropsch), dans lesquelles au moins 70 % des atomes de fer qu'elles contiennent sont présents dans une structure cristalline $Fe_{2,2}C$.

Cependant le coût de ces réactions reste encore important, notamment en raison du coût des particules catalytiques sous forme nanométrique et plus particulièrement les nanoparticules magnétiques. De plus ces nanomatériaux doivent, en général, être manipulés avec précaution.

Un autre problème lié à l'utilisation de nanoparticules est la modification de leurs propriétés de chauffage en raison, d'une part de leur tendance au frittage durant les réactions à haute température, et d'autre part du vieillissement résultant d'un changement de l'ordre chimique dans lesdites nanoparticules (modification de la structure et de la composition chimique locale).

BUTS DE L'INVENTION

Un premier but de l'invention est donc de palier les inconvénients précités en réduisant encore le coût de ces réactions de catalyse hétérogène, tout en maintenant leurs performances réactionnelles.

Un autre but de l'invention est de proposer un procédé permettant de diminuer la part des composants sous forme de particules nanométriques dans le réacteur.

Un autre but de l'invention est de proposer un procédé de catalyse hétérogène présentant un maintien des propriétés de chauffe et des propriétés catalytiques sur de très longues périodes de temps, tout en étant adapté à un fonctionnement par intermittence.

Un autre but de l'invention est de proposer un procédé de catalyse d'une réaction chimique gaz-solide, plus particulièrement d'une réaction d'hydrogénation d'un oxyde de carbone à l'état gazeux, telle qu'une réaction de méthanation.

DESCRIPTION DE L'INVENTION

Dans la recherche de nouvelles économies les inventeurs ont découvert, de manière surprenante, que l'agent chauffant peut ne pas être nécessairement sous forme nanométrique, mais peut être présent dans le réacteur sous forme de poudre micrométrique ou de fils.

A cet effet, la présente invention propose un procédé de catalyse hétérogène d'une réaction d'hydrogénation d'un oxyde de carbone à l'état gazeux, telle qu'une réaction de méthanation mettant en oeuvre, dans un réacteur, du gaz carbonique et du dihydrogène gazeux et au moins un composé solide catalytique apte à catalyser ladite réaction dans une plage de températures T donnée, comprenant la mise en contact dudit réactif gazeux et dudit composé catalytique en présence d'un agent chauffant, et le chauffage de l'agent chauffant à une température comprise dans ladite plage de températures T, le procédé est caractérisé en ce que l'agent chauffant comprend un matériau ferromagnétique sous forme de poudre micrométrique composé de particules ferromagnétiques micrométriques de taille comprises entre 1 $\mu$m et 1000 $\mu$m et/ou de fils à base de fer ou d'un alliage de fer, de préférence présentant un diamètre de fil compris entre 10 micromètres et 1 millimètre, ledit matériau ferromagnétique étant chauffé par induction magnétique au moyen d'un inducteur de champ externe au réacteur, le champ magnétique généré par l'inducteur de champ externe au réacteur ayant une amplitude comprise entre 1mT et 80 mT et une fréquence comprise entre 30 kHz et 500 kHz. Les résultats obtenus avec un tel agent chauffant qui n'est plus nanométrique, mais de taille bien supérieure, sont équivalents à ceux obtenus dans le procédé de WO2014/162099 avec un composant nano-particulaire ferromagnétique.

Selon un premier mode de réalisation de l'invention, lorsqu'il est présent sous forme de poudre, le matériau ferromagnétique est avantageusement composé de particules ferromagnétiques micrométriques de tailles comprises entre 1 $\mu$m et 100 $\mu$m, de préférence comprises entre 1 $\mu$m et 50 $\mu$m, de préférence encore comprises entre 1 $\mu$m et 10 $\mu$m.

Avec de telles particules ferromagnétiques micrométriques, qui ont certes parfois tendance à une agglomération, aucun frittage n'est observé et l'efficacité du chauffage est ainsi maintenue.

En ce qui concerne le composé catalytique mis en oeuvre dans le procédé selon l'invention, ledit composé catalytique comprend un catalyseur de la réaction de catalyse hétérogène se présentant sous forme de particules métalliques disposées sur un support.

Les dites particules métalliques de catalyseur sont avantageusement choisies parmi le manganèse, le fer, le nickel, le cobalt, le cuivre, le zinc, le ruthénium, le rhodium, le palladium, l'iridium, le platine, l'étain ou un alliage comprenant un ou plusieurs de ces métaux.

Lesdites particules métalliques de catalyseur sont disposées à la surface d'un oxyde formant support du catalyseur, tel qu'un oxyde d'au moins un des éléments suivants : silicium, cérium, aluminium, titane, ou zirconium, (par exemple $Al_2O_3$, $SiO_2$, $TiO_2$, $ZrO_2$, $CeO_2$) constituant un ensemble catalyseur-oxyde se présentant sous la forme d'une poudre de taille micrométrique ou nanométrique qui est mélangée au matériau ferromagnétique sous forme de poudre micrométrique. Le mélange de ces poudres (ensemble catalyseur-oxyde avec le matériau ferromagnétique micro-particulaire) crée ainsi un contact intime entre l'agent chauffant et le catalyseur, permettant de démarrer rapidement la réaction de catalyse à la surface du catalyseur.

Selon un second mode de réalisation de l'invention, le support du catalyseur est ledit matériau ferromagnétique se présentant sous forme de fils.

De manière avantageuse, le matériau ferromagnétique se présentant sous forme de fils, supports du catalyseur, peut comprendre, ou être majoritairement constitué, de la paille de fer, renfermant des fils à base de fer ou d'un alliage de fer, de préférence présentant un diamètre de fil compris entre 20 $\mu$m et 500 $\mu$m, de préférence encore entre 50 $\mu$m et 200 $\mu$m.

En effet de manière tout à fait surprenante, la paille de fer, matériau bon marché et aisément disponible, pouvant être acheté dans des magasins de bricolage, s'est révélé être un excellent agent chauffant. Plus particulièrement la paille de fer très fine (dite surfine), présentant un diamètre de fils inférieur au millimètre est à la fois un bon support de catalyseur et efficace pour permettre le chauffage dudit catalyseur par induction magnétique.

Ce matériau est de mise en oeuvre très facile et possède une très longue durée de vie, De plus, il est facilement récupérable et est non polluant.

Le procédé selon l'invention est avantageusement une réaction de synthèse d'hydrocarbure, plus particulièrement la réaction de catalyse hétérogène est.

Le procédé de catalyse hétérogène selon l'invention, réaction d'hydrogénation d'un oxyde de carbone à l'état gazeux, telle qu'une réaction de méthanation à partir de gaz carbonique et de dihydrogène, peut notamment être réalisé avec un champ magnétique généré par l'inducteur de champ externe au réacteur d'une amplitude comprise entre 1 mT et 50 mT et une fréquence comprise entre 50 kHz et 400 kHz, de préférence entre 100 kHz et 300 kHz.

La présente invention décrit également un support de catalyseur pour la mise en oeuvre du procédé de catalyse hétérogène décrit ci-dessus, caractérisé en ce qu'il comprend un matériau ferromagnétique sous forme de fils de diamètres micrométriques, à la surface desquels sont déposées des particules métalliques de catalyseur.

De manière avantageuse, le matériau ferromagnétique est à base de fer ou d'un alliage de fer, de préférence comprenant au moins 50 % massique de fer, de préférence encore au moins 80 % massique de fer.

Ce matériau ferromagnétique peut notamment être composé de paille de fer surfine, comprenant un enchevêtrement de fils composés d'au moins 90 % massique de fer, et dont le diamètre des fils est compris entre 10 $\mu$m et 1 mm, de préférence entre 20 $\mu$m et 500 $\mu$m, de préférence encore entre 50 $\mu$m et 200 $\mu$m.

**Brève description des dessins**

L'invention sera bien comprise à la lecture de la description suivante d'exemples de réalisation, non-limitatifs en référence aux dessins annexés dans lesquels :

[Fig. 1A] est un schéma simplifié partiel d'un réacteur pour la mise en oeuvre du procédé de catalyse hétérogène gaz-solide selon l'invention, sous un flux de gaz ascendant, montrant le positionnement de l'ensemble catalyseur + agent chauffant dans la partie du réacteur tubulaire entourée de l'inducteur de champ magnétique externe,

[Fig. 1B] est un schéma simplifié partiel d'un réacteur pour la mise en oeuvre du procédé de catalyse hétérogène gaz-solide selon l'invention, sous un flux de gaz descendant, montrant le positionnement de l'ensemble catalyseur + agent chauffant dans la partie du réacteur tubulaire entourée de l'inducteur de champ magnétique externe,

[Fig. 2] est un graphique comparant les performances de différents agents chauffant selon l'invention, effectuée sous argon à 100 kHz (taux d'absorption spécifique SAR correspondant à la quantité d'énergie absorbée par unité de masse, exprimée en Watt par gramme de matériau, en fonction de l'intensité de champ magnétique alternatif appliqué, exprimé en mT) : poudre de fer présentant des microparticules de taille de l'ordre de 3-5 $\mu$m, paille de fer fine (diamètre de fils supérieur à 1 mm) et paille de fer surfine (diamètre de fils inférieur à 1 mm, de l'ordre de 100 $\mu$m),

[Fig. 3] est un graphique présentant des résultats d'un procédé de méthanation selon l'invention mettant en oeuvre de la poudre de fer en tant qu'agent chauffant et un catalyseur Ni sur SiRAIOx® (oxyde de silicium et d'aluminium de la société SESAL),

[Fig. 4] est un histogramme montrant les taux de conversion (en %) de $CO_2$ et de $CH_4$ ainsi que la sélectivité en fonction du temps et de la température pour une réaction de méthanation en flux descendant en présence d'un mélange de poudre de fer et de Ni/$CeO_2$,

[Fig. 5] est un histogramme montrant les taux de conversion (en %) de $CO_2$ et de $CH_4$ ainsi que la sélectivité en fonction du temps et de la température pour une réaction de méthanation en flux descendant en présence d'un mélange de paille de fer et de Ni/$CeO_2$,

[Fig. 6] est un histogramme montrant les taux de conversion (en %) de $CO_2$ et de $CH_4$ ainsi que la sélectivité en fonction du temps et de la température pour une réaction de méthanation en flux descendant en présence de nickel sur paille de fer,

[Fig. 7] est un graphique comparatif de l'efficacité énergétique (exprimée en %) en fonction de la température des trois types de lits catalytiques (catalyseur + agent chauffant) testés dans les exemples présentés aux figures 4, 5 et 6.

EXEMPLES

Exemple 1 : Préparation du catalyseur

Préparation du catalyseur sur support oxyde de cérium

Le nickel à 10 % massique sur l'oxyde de Cérium (en abrégé Ni(10wt%)/$CeO_2$) est préparé par décomposition de Ni(COD)$_2$ en présence de $CeO_2$ dans le mésitylène.

Selon un procédé de préparation classique, 1560 mg de Ni(COD)$_2$ sont dissous dans 20 mL de mésitylène puis 3 g de $CeO_2$ sont ajoutés. Le mélange obtenu est chauffé à 150°C sous atmosphère d'Argon pendant 1 heure sous agitation vigoureuse. Ce mélange, initialement blanc laiteux, est noir en fin de réaction. Après décantation, le surnageant translucide est enlevé et les particules obtenues sont lavées trois fois avec 10 mL de toluène. Le toluène est ensuite éliminé sous vide, permettant d'obtenir une poudre épaisse de Ni10wt%/$CeO_2$ (3,5 g) qui est collectée et stockée en boîte à gants. L'analyse par spectroscopie de masse à plasma à couplage inductif (ICP-MS) confirme le chargement de 9 % massique en Nickel (10 % visé) de l'oxyde de cérium. L'observation par microscopie électronique à transmission (MET) et l'analyse EDS montrent la présence de petites particules monodisperses de Nickel (de taille 2-4 nm).

Procédé de préparation de Ni sur SiRAIOx®

Dans une bouteille Fischer-Porter et sous atmosphère inerte, 0,261 g de Ni(COD)$_2$ sont dissous dans 20 mL of mésitylène et 0,500 g of SiRAIOx® sont ajoutés. Le mélange est chauffé à 150°C pendant une heure sous agitation. Après retour à température ambiante, on laisse précipiter la poudre, puis le surnageant est éliminé et la poudre est lavée trois fois avec 10 mL de THF. La poudre est ensuite séchée sous vide et conservée sous atmosphère inerte.

Mélange poudre de fer + Ni/$CeO_2$

2 g de fer en poudre sont mélangés à 1 g de catalyseur nickel déposé sur l'oxyde de cérium préparé précédemment. L'observation au microscope électronique à balayage ainsi que la cartographie EDS permettent de visualiser des grains de poudre de fer de taille de l'ordre de 3-5 μm et de confirmer que le Nickel est bien présent sur l'oxyde de cérium $CeO_2$.

Exemple 2: Préparation du catalyseur sur support paille de fer

Paille de fer surfine (Gerlon, achetée chez Castorama). L'analyse ICP-MS de la paille de fer surfine donne une composition de 94,7 % massique de fer. La cartographie EDS montre la présence de nombreuses impuretés en surface de la paille (potassium, manganèse, silicium principalement). L'observation MEB permet de déterminer le diamètre des fils de la paille de fer surfine utilisée qui est d'environ 100 μm et présente une surface rugueuse et irrégulière.

Le protocole expérimental de dépôt de nickel métallique sur paille de fer surfine (enchevêtrement de fils d'environ 100 μm de diamètre, à 94,7 % massique en fer) est sensiblement le même que sur $CeO_2$. 1560 mg de Ni(COD)$_2$ sont dissous dans 100 mL de mésitylène afin d'immerger totalement la paille de fer (3 g). Après une heure sous vive agitation à 150°C sous argon, le mélange est placé en boite à gant et la solution (de couleur noire) est évacuée. La paille de fer est elle aussi devenue noire. La paille de fer est alors rincée au toluène, puis séchée sous vide pendant 30 minutes et stockée en boîte à gants. L'observation par microscopie électronique à balayage (MEB) et la spectroscopie à rayons X à dispersion d'énergie montrent le dépôt de particules polydisperses de nickel (100 nm-1000 nm) en surface des fils de la paille de fer.

L'analyse par ICP-MS sur trois zones différentes montre des chargements différents en Nickel : 1,23 %, 1,44 % et 1,33 % (pourcentages massiques). Ces différences entre ces chargements sont assez faibles, la surface de la paille semble homogène. Malgré tout, la quantité de nickel déposée est en dessous du pourcentage de 10% massique de Ni visé.

Exemple 3 : réaction de méthanation : mesures de conversion et calcul de la sélectivité

La réaction de méthanation

$$[Chem. 1] \quad CO_2 \cdot + \cdot 4 \cdot H_2 \cdot \rightarrow \cdot CH_4 \cdot + \cdot 2 \cdot H_2O \cdot$$

qui est une combinaison de

[Chem. 2]   ·CO$_2$+·H$_2$·↔·CO+·H$_2$O·

et de

[Chem. 3]   CO·+·3·H$_2$→·CH$_4$·+·H$_2$O·

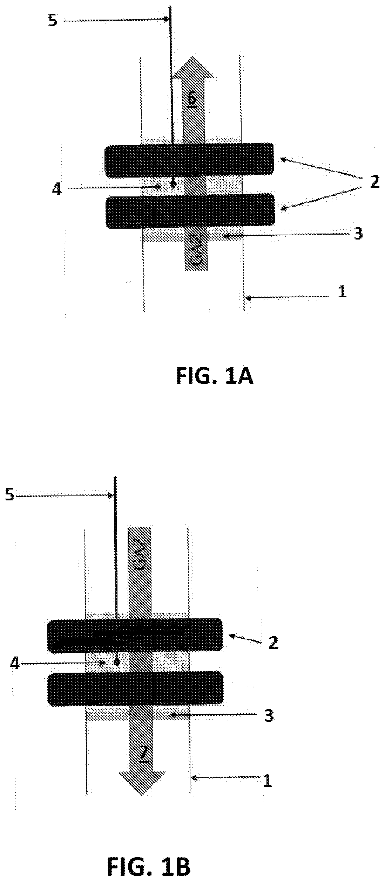

est réalisée dans un réacteur 1 continu tubulaire à lit fixe et en quartz (Avitec) (diamètre interne : 1 cm avec une hauteur de lit catalytique 4, dépendant de l'élément chauffant, d'environ 2 cm, reposant sur verre fritté 3) (cf figure 1) ; le courant gazeux peut être à flux ascendant 6 (Fig.1A) ou à flux descendant 7 (Fig.1B)). La bobine 2 (de la société Five Celes) utilisée est un solénoïde de diamètre intérieur 40 mm et de 40 mm de hauteur constituant l'inducteur de champ magnétique externe relié à un générateur. Sa fréquence de résonance est de 300 kHz avec un champ magnétique variant entre 10 et 60 mT. La bobine 2 est refroidie à l'eau.

Les mesures de taux de conversion et de la sélectivité en fonction de la température, sont effectuées avec asservissement en température du générateur associé à la bobine 2. A cet effet, une sonde de température 5 reliée au générateur est plongée dans le lit catalytique (ensemble agent chauffant + catalyseur). Le générateur envoie un champ magnétique pour atteindre la température fixée et n'envoie ensuite que des impulsions pour maintenir cette température. La réaction se fait à pression atmosphérique et à une température variant entre 200 et 400°C. Le réacteur 1 est alimenté en H$_2$ et en CO$_2$ dont le débit est contrôlé par un débitmètre (débitmètre Brooks) et piloté par un logiciel Lab View. Les proportions sont les suivantes : un débit constant global de 25 mL/min comprend 20 mL/min d'H$_2$ et 5 mL/min de CO$_2$. L'alimentation se fait en tête du réacteur, l'eau formée est condensée en pied de réacteur (sans condenseur) et est récupérée dans un ballon. Le méthane formé et les gaz restants (CO$_2$ et H$_2$) ainsi que le CO sont envoyés dans une colonne de chromatographie en phase gazeuse (colonne GC Perkin Elmer, Clarus 580). La conversion du CO$_2$, la sélectivité du CH$_4$ et le rendement du CO et du CH$_4$ sont calculés selon les équations suivantes

[Math. 2]

$$X(CO_2) = \text{Conversion } CO_2 = \frac{FC(CO) \times A(CO) + FC(CH_4) \times A(CH_4)}{FC(CO) \times A(CO) + FC(CH_4) \times A(CH_4) + A(CO_2)}$$

$$Y(CO) = \text{Rendement } CO = \frac{FC(CO) \times A(CO)}{FC(CO) \times A(CO) + FC(CH_4) \times A(CH_4) + A(CO_2)}$$

$$Y(CH_4) = \text{Rendement } CH_4 = \frac{FC(CH_4) \times A(CH_4)}{FC(CO) \times A(CO) + FC(CH_4) \times A(CH_4) + A(CO_2)}$$

$$S(CH_4) = \text{Sélectivité } CH_4 = \frac{FC(CH_4) \times A(CH_4)}{FC(CO) \times A(CO) + FC(CH_4) \times A(CH_4)}$$

Avec $FC(CO) = 1{,}61$ et $FC(CH_4) = 1{,}71$.

FC est le facteur de réponse pour chaque réactif selon un suivi de réaction par chromatographie en phase gazeuse,
A est l'aire du pic mesuré en chromatographie.

Mesures de l'efficacité énergétique :

Des mesures d'efficacité énergétique sont effectuées en parallèle des mesures de conversion et de sélectivité de la réaction de méthanation. Les données de consommation électrique de la bobine 2 sont récupérées au moyen d'un logiciel développé au laboratoire. L'efficacité énergétique se calcule alors de la façon suivante

[Math. 2]

$$\eta_{therm-NRJ} = \frac{Y_{CH4} \cdot D_{m,CH4} \cdot PCS_{CH4}}{D_{m,H2} \cdot PCS_{H2} + E_{bobine}}$$

PCS (pouvoir calorique supérieur) représente la quantité d'énergie libérée par la combustion d'1mg de gaz,

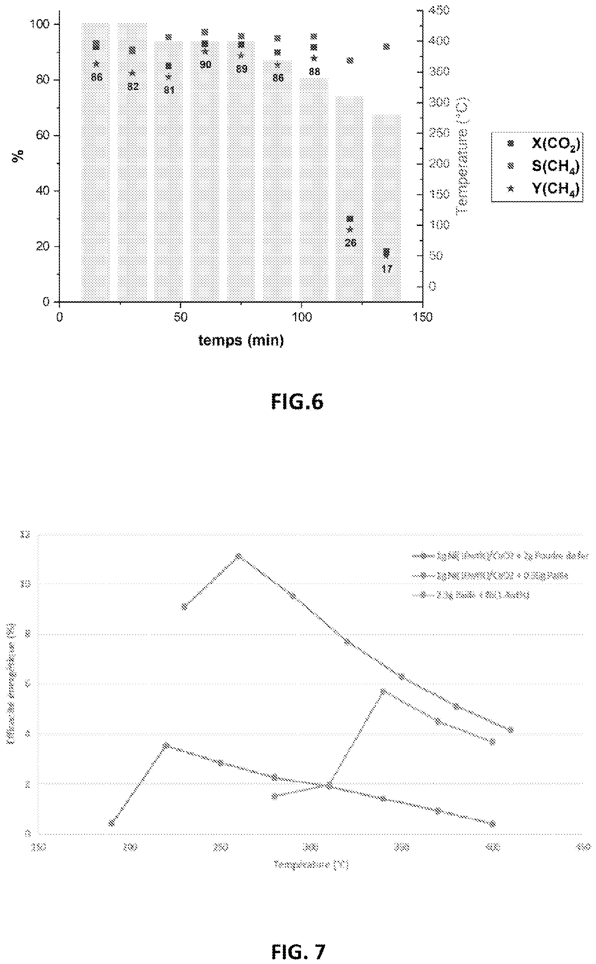

Les valeurs données par la littérature sont $PCS_{H2}$ = 141,9 MJ/kg et $PCS_{CH4}$ = 55,5 MJ/kg,
$Y_{CH4}$ étant le rendement de la réaction en $CH_4$,
$D_{mi}$ le débit massique du produit i,
$E_{bobine}$ correspond à l'énergie consommée par l'inducteur pour fonctionner (à savoir, générer le champ magnétique et refroidir le système),
L'efficacité énergétique est exprimée en % sur la figure 7.

Exemple 4 : Comparaison de différents agents chauffants

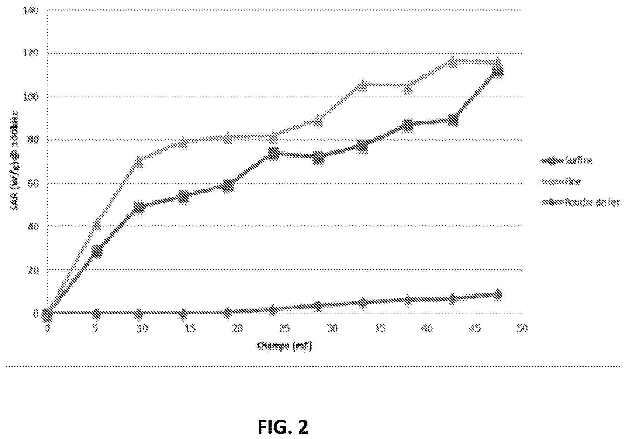

La poudre de fer, la paille de fer fine et de la paille de fer surfine ont été comparées. Les mesures du taux d'absorption spécifique (SAR) (correspondant à la quantité d'énergie absorbée par unité de masse, exprimée en Watt par gramme de matériau), en fonction de l'intensité de champ magnétique alternatif appliqué, exprimé en mT) ont été effectuées à 100 kHz sous argon. Les résultats sont regroupés sur la figure 2.

Ces résultats diffèrent notablement de ceux obtenus dans la publication récente de Kale et al, Iron carbide or iron carbide/cobalt nanoparticles for magnetically-induced CO2 hydrogénation over Ni/SiRAIOx catalysts, Catal. Sci. Technol., 2019, 9, 2601., qui rapporte pour les nanoparticules de FeC des valeurs de SAR comprises entre 1100 et 2100 W/g à 100 kHz. La figure 2 montre que pour un matériau ferromagnétique micro-particulaire tel que la poudre de fer ou la paille de fer ces valeurs sont 10 à 20 fois inférieures.

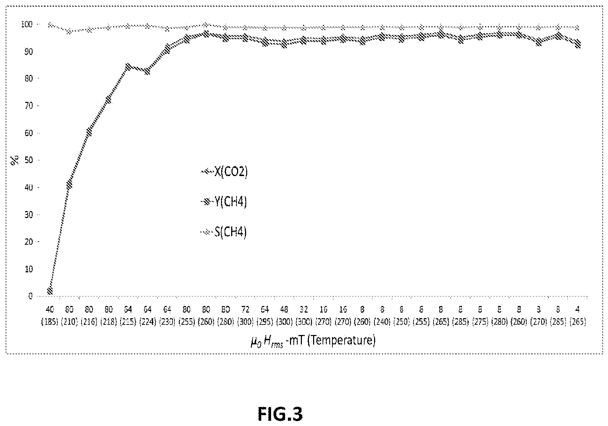

On pourrait alors s'attendre à devoir apporter à la poudre de fer micro-particulaire et à la paille de fer un champ plus important que pour les nanoparticules. Mais les résultats de la figure 3 montrent que ce n'est pas le cas. Pour les nanoparticules de carbure de fer il faut apporter un champ d'environ 48 mT pour atteindre un rendement proche de 90%. Avec la poudre de fer, après lancement de la réaction, un champ de seulement 8 mT est nécessaire. La particularité de la poudre de fer et de la paille de fer réside dans les courants de Foucault qui entrent en jeu et entrainent une diminution du champ magnétique pour échauffer le matériau.

La poudre de fer et la paille de fer micrométriques constituent donc des matériaux ferromagnétiques intéressants pour chauffer in situ par induction magnétique les réacteurs mettant en oeuvre des réactions catalytiques gaz-solide telles que les réactions de méthanation à partir de gaz carbonique et de dihydrogène, ce qui est présenté dans les exemples qui suivent.

Exemple 5 : mélange de poudres de fer et de catalyseur

Le lit catalytique est constitué de particules de nickel sur oxyde de cérium : Ni : 0,09 g / $CeO_2$ : 0,91 g, mélangées à 2 g de poudre de fer. Le flux de gaz est descendant, à un débit constant de 20 mL/min de $H_2$ et 5 mL/min de $CO_2$.

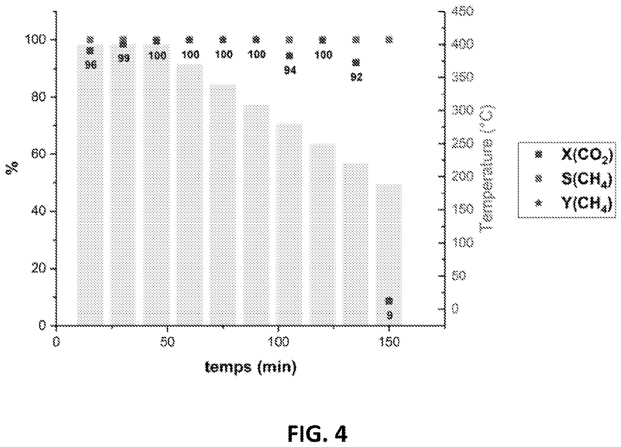

Les résultats des taux de conversion en $CO_2$ et en $CH_4$ sont présentés sur la figure 4. Cet ensemble de poudres (poudre fer + Ni/$CeO_2$) permet d'obtenir des rendements (Y($CH_4$)) très satisfaisants, atteignant 100% aux températures de 300-350°C.

Exemple 6 : mélange de paille de fer et de catalyseur Ni/$CeO_2$

Le lit catalytique est constitué de particules de nickel déposées sur oxyde de cérium : Ni : 0,09 g / $CeO_2$ : 0,91 g et de 0,35 g de paille de fer (surfine). Le flux de gaz est descendant, à un débit constant de 20 mL/min de $H_2$ et 5 mL/min de $CO_2$.

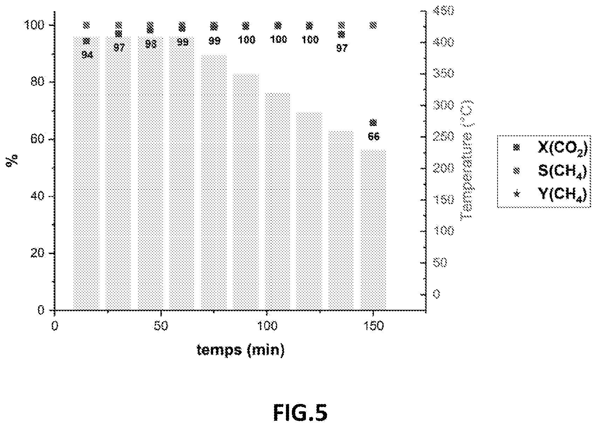

Les résultats des taux de conversion en $CO_2$ et en $CH_4$ sont présentés sur la figure 5. Cet ensemble paille de fer + Ni/$CeO_2$ permet également d'obtenir des rendements (Y($CH_4$)) très satisfaisants, atteignant 100% aux températures de 300-350°C.

Exemple 7 : catalyseur Ni déposé sur paille de fer

Le lit catalytique est constitué de particules de nickel: Ni : 0,03 g déposées sur 2,27 g de paille de fer (surfine).

Le flux de gaz est descendant, à un débit constant de 20 mL/min de $H_2$ et 5 mL/min de $CO_2$.

Les résultats des taux de conversion en $CO_2$ et en $CH_4$ sont présentés sur la figure 6. Le rendement maximal ($Y(CH_4)$) est de 90% à 400°C. Ce résultat est très encourageant sachant que ce système est le plus simple à mettre en oeuvre.

Exemple 8 : efficacité énergétique

Les calculs d'efficacité énergétique des trois exemples précédents (exemples 5, 6 et 7) regroupés sur la figure 7 montrent qu'il faut apporter moins d'énergie au système paille de fer qu'au système poudre de fer pour atteindre une même température. Cette différence entre poudre et paille s'observe surtout avec le système paille de fer + $Ni/CeO_2$. L'efficacité énergétique de la paille de fer + Ni n'est pas aussi bonne car il y a davantage de paille à chauffer et donc davantage d'énergie à apporter pour une même quantité de méthane produite. Dans l'exemple présenté il a fallu introduire une quantité supérieure de paille de fer, car très peu de nickel y avait été déposé, pour atteindre un rendement intéressant (90%).

**Revendications**

1. Procédé de catalyse hétérogène d'une réaction d'hydrogénation d'un oxyde de carbone à l'état gazeux, telle qu'une réaction de méthanation mettant en oeuvre, dans un réacteur (1), du gaz carbonique et du dihydrogène gazeux et au moins un composé solide catalytique apte à catalyser ladite réaction dans une plage de températures T donnée, comprenant la mise en contact dudit réactif gazeux et dudit composé catalytique en présence d'un agent chauffant, et le chauffage de l'agent chauffant à une température comprise dans ladite plage de températures T, **caractérisé en ce que** l'agent chauffant comprend un matériau ferromagnétique sous forme de poudre micrométrique composé de particules ferromagnétiques micrométriques de tailles comprises entre 1 μm et 1000 μm et/ou de fils à base de fer ou d'un alliage de fer, de préférence présentant un diamètre de fil compris entre 10 micromètres et 1 millimètre, ledit matériau ferromagnétique étant chauffé par induction magnétique au moyen d'un inducteur de champ externe au réacteur (1), le champ magnétique généré par l'inducteur de champ externe au réacteur ayant une amplitude comprise entre 1mT et 80 mT et une fréquence comprise entre 30 kHz et 500 kHz.

2. Procédé selon la revendication 1, **caractérisé en ce que** le matériau ferromagnétique sous forme de poudre est composé de particules ferromagnétiques micrométriques de tailles comprises entre 1 μm et 100 μm.

3. Procédé selon l'une des revendications 1 ou 2, **caractérisé en ce que** le matériau ferromagnétique sous forme de poudre est composé de particules ferromagnétiques, de tailles comprises entre 1 μm et 50 μm, de préférence comprises entre 1 μm et 10 μm.

4. Procédé selon l'une des revendications 1 à 3, **caractérisé en ce que** le composé catalytique comprend un catalyseur de la réaction de catalyse hétérogène se présentant sous forme de particules métalliques disposées sur un support.

5. Procédé selon la revendication 4, **caractérisé en ce que** lesdites particules métalliques de catalyseur sont choisies parmi le manganèse, le fer, le nickel, le cobalt, le cuivre, le zinc, le ruthénium, le rhodium, le palladium, l'iridium, le platine, l'étain ou un alliage comprenant un ou plusieurs de ces métaux.

6. Procédé selon l'une des revendications 4 ou 5, **caractérisé en ce que** les particules métalliques de catalyseur sont disposées à la surface d'un oxyde formant support du catalyseur, tel qu'un oxyde d'au moins un des éléments suivants : silicium, cérium, aluminium, titane, ou zirconium, constituant un ensemble catalyseur-oxyde se présentant sous la forme d'une poudre qui est mélangée au matériau ferromagnétique sous forme de poudre.

7. Procédé selon l'une des revendications 4 à 6, **caractérisé en ce que** le support du catalyseur est ledit matériau ferromagnétique se présentant sous forme de fils.

8. Procédé selon la revendication 7, **caractérisé en ce que** le matériau ferromagnétique se présentant sous forme de fils, supports du catalyseur, comprend de la paille de fer, renfermant des fils à base de fer ou d'un alliage de fer, de préférence présentant un diamètre de fil compris entre 20 μm et 500 μm, de préférence entre 50 μm et 200 μm.

9. Procédé selon l'une quelconque des revendications précédentes, **caractérisé en ce que** le champ magnétique généré par l'inducteur de champ externe au réacteur a une amplitude comprise entre 1 mT et 50 mT.

10. Procédé selon l'une quelconque des revendications précédentes, **caractérisé en ce que** le champ magnétique généré par l'inducteur de champ externe au réacteur a une fréquence comprise entre 50 kHz et 400 kHz, de préférence entre 100 kHz et 300 kHz.

**Patentansprüche**

1. Verfahren zur heterogenen Katalyse einer Hydrierungsreaktion eines Kohlenmonoxids in gasförmigem Zustand, wie z. B. einer Methanisierungsreaktion, die, in einen Reaktor (1), Kohlendioxid und gasförmigen Wasserstoff und mindestens eine feste katalytische Verbindung, die geeignet ist, um die Reaktion in einem gegebenen Temperaturbereich T zu katalysieren, einführt, umfassend das In-Kontakt-Bringen des gasförmigen Reaktanten und der katalytischen Verbindung in Anwesenheit eines Heizmittels und das Erhitzen des Heizmittels auf eine Temperatur, die im Temperaturbereich T liegt, **dadurch gekennzeichnet, dass** das Heizmittel ein ferromagnetisches Material in Form eines mikrometrischen Pulvers umfasst, zusammengesetzt aus mikrometrischen ferromagnetischen Partikeln mit Größen im Bereich zwischen 1 μm und 1000 μm und/oder Drähten auf der Grundlage von Eisen oder einer Eisenlegierung, vorzugsweise mit einem Drahtdurchmesser im Bereich zwischen 10 Mikrometern und 1 Millimeter, wobei das ferromagnetische Material durch magnetische Induktion mit Hilfe eines Feldinduktors außerhalb des Reaktors (1) erhitzt wird, wobei das Magnetfeld, erzeugt durch den Feldinduktor außerhalb des Reaktors eine Amplitude, die im Bereich zwischen 1 mT und 80 mT liegt, und eine Frequenz aufweist, die im Bereich zwischen 30 kHz und 500 kHz liegt.

2. Verfahren nach Anspruch 1, **dadurch gekennzeichnet, dass** das ferromagnetische Material in Form eines Pulvers aus mikrometrischen ferromagnetischen Partikeln mit Größen im Bereich zwischen 1 μm bis 100 μm zusammengesetzt ist.

3. Verfahren nach einem der Ansprüche 1 oder 2, **dadurch gekennzeichnet, dass** das ferromagnetische Material in Form eines Pulvers aus ferromagnetischen Partikeln mit Größen im Bereich zwischen 1 μm und 50 μm der vorzugsweise im Bereich zwischen 1 μm und 10 μm zusammengesetzt ist.

4. Verfahren nach einem der Ansprüche 1 bis 3, **dadurch gekennzeichnet, dass** die katalytische Verbindung einen Katalysator der heterogenen Katalysereaktion umfasst, die die Form von metallischen Partikeln aufweist, die auf einem Träger angeordnet sind.

5. Verfahren nach Anspruch 4, **dadurch gekennzeichnet, dass** die metallischen Katalysatorpartikel ausgewählt sind aus Mangan, Eisen, Nickel, Kobalt, Kupfer, Zink, Ruthenium, Rhodium, Palladium, iridium, Platin, Zinn oder einer Legierung, umfassend eines oder mehrere dieser Metalle.

6. Verfahren nach einem der Ansprüche 4 oder 5, **dadurch gekennzeichnet, dass** die metallischen Katalysatorpartikel auf der Fläche eines Oxids angeordnet sind, das einen Katalysatorträger bildet, wie z. B. eines Oxids mindestens eines der folgenden Elemente: Silizium, Cer, Aluminium, Titan oder Zirkonium, die eine Einheit Katalysator-Oxid bilden, die die Form eines Pulvers aufweist, das mit dem ferromagnetischen Material in Form eines Pulvers gemischt ist.

7. Verfahren nach einem der Ansprüche 4 bis 6, **dadurch gekennzeichnet, dass** der Katalysatorträger das ferromagnetische Material ist, das die Form von Drähten aufweist.

8. Verfahren nach Anspruch 7, **dadurch gekennzeichnet, dass** das ferromagnetische Material, das die Form von Drähten aufweist, Stahlwolle aufweist, die Drähte auf der Grundlage von Eisen oder einer Eisenlegierung einschließt, die vorzugsweise mit einem Drahtdurchmesser aufweisen, der im Bereich zwischen 20 μm und 500 μm, vorzugsweise zwischen 50 μm und 200 μm liegt.

9. Verfahren nach einem der vorhergehenden Ansprüche, **dadurch gekennzeichnet, dass** das Magnetfeld, erzeugt durch den Feldinduktor, außerhalb des Reaktors eine Amplitude aufweist, die im Bereich zwischen 1 mT und 50 mT liegt.

10. Verfahren nach einem der vorhergehenden Ansprüche, **dadurch gekennzeichnet, dass** das Magnetfeld, erzeugt durch den Feldinduktor, außerhalb des Reaktors eine Frequenz aufweist, die im Bereich zwischen 50 kHz und 400 kHz, vorzugsweise zwischen 100 kHz und 300 kHz liegt.

**Claims**

1. A method for heterogeneous catalysis of a hydrogenation reaction of a carbon oxide in the gaseous state, such as a methanation reaction employing, in a reactor (1), carbon dioxide and dihydrogen gas and at least one solid catalytic compound capable of catalysing said reaction over a given range of temperatures T, comprising the contacting of said gaseous reactant and said catalytic compound in the presence of a heating agent, and heating the heating agent to a temperature lying in said range of temperatures T, **characterized in that** the heating agent comprises a ferromagnetic material in micrometric powder form composed of micrometric ferromagnetic particles of sizes between 1 $\mu$m and 1000 $\mu$m and/or iron- or iron alloy-based wire preferably having a wire diameter of between 10 micrometres and 1 millimetre, said ferromagnetic material being heated by magnetic induction by means of a field inductor external to the reactor (1), the magnetic field generated by the field inductor external to the reactor having an amplitude of between 1mT and 80 mT and a frequency of between 30 kHz and 500 kHz.

2. The method according to claim 1, **characterized in that** the ferromagnetic material in powder form is composed of micrometric ferromagnetic particles of sizes between 1 $\mu$m and 100 $\mu$m.

3. The method according to one of claims 1 or 2, **characterized in that** the ferromagnetic material in powder form is composed of ferromagnetic particles of sizes between 1 $\mu$m and 50 $\mu$m, preferably between 1 $\mu$m and 10 $\mu$m.

4. The method according to one of claims 1 to 3, **characterized in that** the catalytic compound comprises a catalyst of the heterogeneous catalysis reaction in the form of metal particles arranged on a support.

5. The method according to claim 4, **characterized in that** said metal catalyst particles are selected from among manganese, iron, nickel, cobalt, copper, zinc, ruthenium, rhodium, palladium, iridium, platinum, tin or an alloy comprising one or more of these metals.

6. The method according to one of claims 4 or 5, **characterized in that** the metal catalyst particles are arranged on the surface of an oxide forming a catalyst support, such as an oxide of at least one of the following elements: silicon, cerium, aluminium, titanium or zirconium, forming a catalyst-oxide assembly in the form of a powder which is mixed with the ferromagnetic material in powder form.

7. The method according to one of claims 4 to 6, **characterized in that** the catalyst support is said ferromagnetic material in wire form.

8. The method according to claim 7, **characterized in that** the ferromagnetic material in wire form, the catalyst support, comprises wire wool containing iron- or iron alloy-based wires, preferably having a wire diameter of between 20 $\mu$m and 500 $\mu$m, preferably between 50 $\mu$m and 200 $\mu$m.

9. The method according to any of the preceding claims, **characterized in that** the magnetic field generated by the field inductor external to the reactor has an amplitude of between 1 mT and 50 mT.

10. The method according to any of the preceding clams, **characterized in that** the magnetic field generated by the field inductor external to the reactor has a frequency of between 50 kHz and 400 kHz, preferably between 100 kHz and 300 kHz.

**FIG. 1A**

**FIG. 1B**

**FIG. 2**

FIG.3

**FIG. 4**

**FIG.5**

**FIG.6**

**FIG. 7**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 2014162099 A **[0003] [0016]**
- US 2017348454 A1 **[0004]**
- US 4359379 A **[0005]**
- US 2019144376 A **[0006]**
- WO 2015140620 A1 **[0007]**
- FR 3045412 A1 **[0008]**

### Littérature non-brevet citée dans la description

- **KALE et al.** Iron carbide or iron carbide/cobalt nanoparticles for magnetically-induced CO2 hydrogénation over Ni/SiRAIOx catalysts. *Catal. Sci. Technol.,* 2019, vol. 9, 2601 **[0045]**